# EUROPEAN PATENT SPECIFICATION

(11) **EP 3 207 879 B1**
(45) Date of publication and mention of the grant of the patent: **29.04.2020**
(21) Application number: 16382067.3
(22) Date of filing: 19.02.2016
(51) Int. Cl.: A61B 10/02

(54) **DEVICE FOR THE SELF-COLLECTION OF CELLS FROM THE CERVIX**
VORRICHTUNG ZUR SELBSTENTNAHME VON ZELLEN AUS DEM UTERUSHALS
DISPOSITIF POUR LA COLLECTE AUTOMATIQUE DE CELLULES DU COL DE L'UTÉRUS

(43) Date of publication of application: 23.08.2017
(73) Proprietor: Self Test Technologies S.L., 07013 Palma de Mallorca (ES)
(72) Inventor: Rey Lázaro, Maximino, 07340 Puerto de Alcudia (ES)
(74) Representative: Carlos Hernando, Borja

(56) References cited:
- WO-A1-97/24071
- WO-A1-2011/021931
- WO-A2-2006/116018
- US-A- 6 036 658
- US-A1- 2011 224 575
- US-A1- 2013 066 233

## Description

### OBJECT OF THE INVENTION

The present invention, a device for the self-collection of cells from the cervix, relates to a device designed for allowing women themselves to carry out the collection of cells from the cervix, with the aim of being able to diagnose women with precancerous lesions or cancer, who, for different reasons, lack the possibility of going to the gynecologist or refuse a pelvic examination. To this end, the device is marketed packaged in a package where it is already prepared and sterilized to be used directly without any previous operation, beyond the removal of a cap for covering and protecting the area which is introduced into the vagina and where the sample is collected and which, once collected, serves for protecting said area once again.

The application field of the present invention is framed within the sector of the industry dedicated to the manufacture of healthcare products and devices, being focused within the scope of those intended for the autonomous collection of biological samples for single use.

### BACKGROUND OF THE INVENTION

As is known, early diagnosis is the most powerful weapon for fighting cancer. Thus, in the case of women, gynecological checks are very important for preventing or screening cervical cancer.

However, although it would be desirable for all women to regularly complete said checks, which would have a positive impact, reducing the global occurrence of cervical cancer and, consequently, the mortality associated with said cancer, many women, for different reasons, do not regularly attend the gynecologist or do not go directly because they refuse a pelvic examination.

The essential objective of the present invention is thus to develop an easy to use device which allows women, who so desire, to be able to collect the samples themselves in order to carry out the corresponding analyses, avoiding the need to attend the gynecologist or to be subjected to an examination, a second objective being that the device allows said samples to be appropriately contained and protected for the dispatch thereof to the relevant laboratory without undergoing changes and, lastly, it is also an objective of the invention for said device to be for single use in order to guarantee hygiene and a low economic cost. WO 2006/116018 A2 discloses a device for the self-collection of cells from the cervix according to the preamble of claim 1.

### DESCRIPTION OF THE INVENTION

The device for the self-collection of cells from the cervix is a device intended for the use thereof by women for carrying out the collection of cells themselves from their own cervix and being able to dispatch it to the laboratory.

The objective of the present invention is a device according to claim 1. Specifically, the device for the self-collection of cells from the cervix comprises an interior cannula which comprises a plunger with a handle in the proximal area and a distal collection area with a hemispherical point which is referred to here as the thimble and one exterior sheath which is passed through by the cannula allowing, by means of the rotation of the same once it is situated in the vagina, the removal of the collection area through the distal end of the sheath, at the same time as the rotation thereof to carry out the collection of cells. In order to achieve said rotation and displace the sheath with respect to the cannula, rotation means are provided which allow the displacement of the exterior sheath between two positions, a first position in which the collection thimble of the cannula (2) is hidden under the sheath and a second position in which the collection thimble is outside of the sheath.

Preferably, the collection area or thimble forms part of the piece itself that forms the cannula, consisting of a cylindrical piece and this collection area is defined by an embossed surface of the distal end of said piece, preferably a grooved surface formed by a series of grooves in the form of parallel lines arranged in the longitudinal direction, that is to say, parallel to the axial axis of said cannula which converge radially at the hemispherical end thereof. This part of the device is the part that carries out the physical collection of the cells, and thus, has been designed seeking minimum invasiveness to the vaginal wall with which it must enter into contact for the collection.

The cannula is also preferably a low-density polyethylene piece for healthcare use, although other materials suitable for said function can be used, other such materials may be alternative biocompatible plastics for healthcare use. Said cannula has a configuration designed for the impulsion and guiding of the collection thimble through the sheath, acting as a support for said thimble which, as had been indicated, constitutes the collection area of the sample of cells. For said rotational impulsion and guiding of the thimble, the cannula, in the part of the same that forms the plunger which is displaced within the sheath, has two protrusions which, located on opposing sides of the surface thereof, run inserted in respective helicoidal channels provided for this purpose in the internal wall of said sheath. In this way, when the cannula is made to rotate inside the sheath, the displacement of the cannula is produced, the collection thimble emerging through the hole of the end of the sheath when the sheath descends with respect to the cannula or when the cannula ascends with respect to the sheath.

Furthermore and in order to facilitate said rotational operation, the cannula has, on the proximal part thereof, that is to say, the part opposed to that of the collection thimble, a grip handle or area.

The sheath is, in turn, the piece which allows the entry of the assembly inside the vagina and guides the rotation of the cannula, said sheath is also preferably formed by a piece of plastic material, preferably low-density polyethylene for healthcare use although other materials suitable for said function can be used, other such materials may be alternative biocompatible plastics for healthcare use. To this end, it is a preferably hollow piece with a cylindrical geometry and an open hemispherical point to allow the exit of the collection thimble. Furthermore, in the proximal area, the sheath has a fin intended to allow the easy gripping thereof by means of two fingers which allows comfortable handling, gripping and actuation of the device with one hand, while the other rotates the cannula.

The device also envisaged the existence of a cap which covers the distal part of the sheath in the position thereof prior to use, which is the position in which the device is distributed for the marketing thereof and which, for the use of the same, is removed, replacing it once the collection of the sample has taken place, this time locating it over the collection thimble since this will have been removed from the sheath.

Lastly, it should be indicated that the device is preferably presented and marketed packaged in a kit which comprises, the device protected with the cap already prepared and sterilized. That is to say, with the cannula installed inside the sheath in the initial position and with the cap press-fitted on the distal end of the sheath. In this position, the assembly is stable and can be handled as an assembly for the sterilization and packaging thereof. Optionally, the marketing kit also comprises a package with a label for the identification and dispatch thereof to the laboratory.

For the use thereof, the following steps should be followed:
1- INSERTION: Once the device has been removed from the sterile package thereof, it is found in the initial delivery position and is already prepared to be introduced into the vagina, without any previous operation, with the distal end thereof which is the narrowest side, approximately up to a mark that will be indicated on the same for such purpose, in particular on the exterior of the sheath. The cap, which covers the distal portion where the collection area is found, is removed and, for the introduction thereof, the woman holds it with one hand at the rear end thereof using the other hand to assist in the operation, by separating the labia as if this were a vaginal tampon.
2.- SLIDING and COLLECTION: Once the product has been introduced into the vagina, by means of light pressure, the sheath can be pushed towards the exterior of the body thereof, leaving the collection thimble, which exits the sheath, within the vagina. In this way, the thimble is in contact with the cervix. With the rotation of the cannula as it exits the sheath and therefore with the rotation of the collection thimble, the linear grooved surface of the thimble will remove cells from the cervix which will be adhered to the surface of said collection thimble. The rotation is easily carried out by gripping the product by the part which is visible on the exterior of the vagina. Once this has been done, the product can be removed from the vagina. In the preferred embodiment, the removal of the thimble from the sheath and the rotation of the same is carried out at the same time as rotating the cannula inside the sheath.
3.- CONTAINER FOR THE DEVICE ENCLOSED IN THE PACKAGE: Once the device has been removed from the vagina, the collection thimble is covered with the cap which initially covers the sheath, thereby preserving the collected cells.

Once the device has been suitably identified, it is ready for the dispatch thereof to the laboratory responsible for carrying out the analysis of the cytological sample.

### DESCRIPTION OF THE DRAWINGS

In order to complete the description of the invention and with the aim of facilitating the understanding of the characteristics of the same, figures are included in the present specification, as an integral part of the same, which depict the invention in an illustrative and non-limiting manner.
Figure 1 shows an elevation view of the cutting of an example of the device object of the invention, the principal parts and elements which it comprises being observed as well as the configuration of each one.

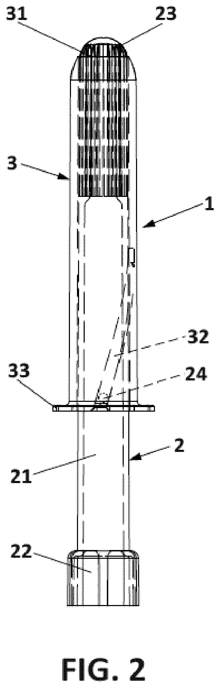

Figure 2 shows an elevation view of the cannula and the sheath coupled to each other, the interior channels of the sheath for directing the circular removal movement of the collection thimble from the cannula being depicted by means of discontinued dashed lines.
Figures 3, 4, 5 and 6 show two elevation views of the different phases of functioning of the device.

### PREFERRED EMBODIMENT OF THE INVENTION

In view of the mentioned figures and according to the numeration adopted below, a description of an exemplary embodiment of the present invention is given.

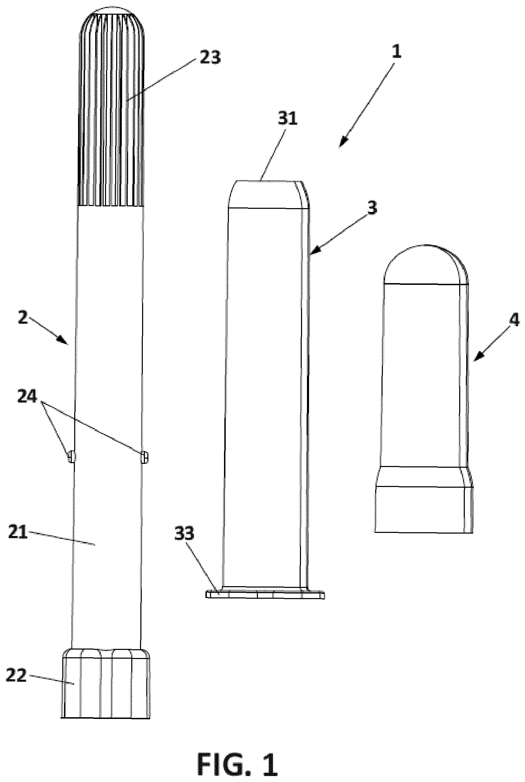

Thus, in terms of Figure 1, it can be observed how the device (1) object of the invention comprises at least: one interior cannula (2), formed by a plunger (21) which preferably has a handle (22) in the proximal area and a collection thimble (23) in the distal or opposing area; and one exterior sheath (3) which is passed through by the interior cannula (2), allowing, owing to rotation means, the sliding thereof and the removal, at least of the collection thimble (23) through the hole (31) of the distal end thereof as well as the rotation thereof for carrying out the collection of cells.

The device (1) preferably also has a protective cap (4) which covers the distal area of the sheath (3) in the initial delivery position thereof and the collection thimble (23) of the interior cannula (2) once used. Said collection thimble (23) is defined by an embossed surface of the distal end of the piece which constitutes the interior cannula (2), a surface which is preferably grooved and formed by parallel longitudinal grooves which converge radially on the hemispherical end thereof.

The interior cannula (2) and the sheath (3) are structured to be coupled and carry out the displacement which simultaneously determines the removal of the collection thimble (23) outside of the sheath (3) and the rotation thereof, for which the interior sheath (2) has two protrusions (24) on opposing sides of the surface of the plunger (21) which fit in respective helicoidal channels (32) provided on the interior wall of the sheath (3), which guide the rotation and displacement of the cannula inside the sheath (3), or vice versa, when the sheath (3) is held and the handle (22) of the cannula (2) made to rotate.

In order to facilitate said holding of the sheath (3), it has, in the proximal area thereof, a gripping fin (33) which allows comfortable handling.

Lastly, it should be indicated that at least the interior cannula (2) and the sheath (3) are made of plastic material, preferably low-density polyethylene for healthcare use.

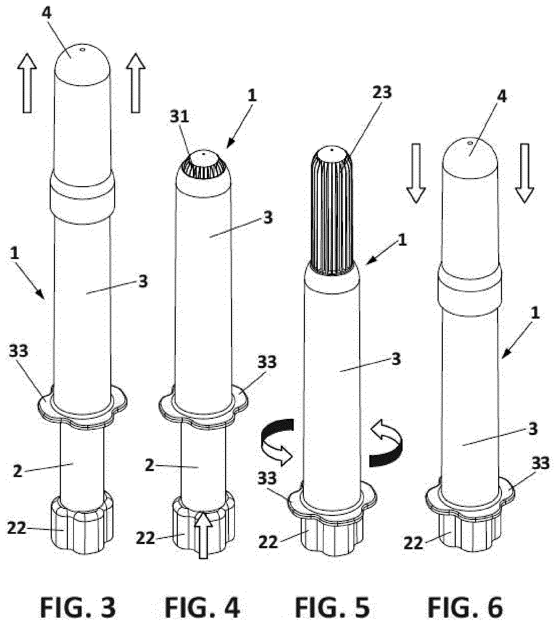

In Figures 3 to 6, the phases of operation of the device are observed. Thus, firstly (Figure 3), the cap (4), preferably press-fitted on the sheath (3), is removed, although it could also be threaded, with the cannula internally incorporated in the initial delivery position. Once the cap (4) has been removed (Figure 4) and the distal end of the assembly of sheath (3) and cannula (2) placed in the vagina, the rotation of the interior cannula (2) is carried out, holding the sheath (3) by the fin (33) and making the handle (22) of said cannula rotate, a movement which produces the sliding and rotation of the interior cannula, causing the exit of the collection thimble (23) outside of the sheath (3) (Figure 5) and the rotation thereof, touching the walls of the uterus, causing the cells in the interior of the vagina to be deposited on the grooved surface. Lastly, the device is removed from the vagina and the collection thimble (23) is covered with the protective cap (4) (Figure 6).

## Claims

1. A device for the self-collection of cells from the cervix, comprising at least:
- one interior cannula (2) which comprises a plunger (21) at a proximal end and a collection thimble (23) at the distal end thereof; and
- one exterior sheath (3) which is passed through by the interior cannula (2), and
- rotation means (32) between the interior cannula (2) and the exterior sheath (3) which allow the rotation and sliding of the exterior sheath (3) on the interior cannula (2),
**characterized in that** that the rotation means comprise two protrusions (24) on opposing sides of the surface of the plunger (21) of the cannula (2) which fit in respective helicoidal channels (32) provided on the interior wall of the sheath (3)

2. The device according to claim 1, **characterized in that** the collection thimble (23) is defined by an embossed surface of the distal end of the piece which constitutes the interior cannula (2).

3. The device according to claim 2, **characterized in that** the embossed surface of the collection thimble (23) is a grooved surface formed by parallel longitudinal grooves which converge radially on the hemispherical end thereof.

4. The device according to the preceding claims, **characterized in that** the rotation means allow for the displacement of the exterior sheath (3) between two positions, a first position in which the collection thimble (23) of the cannula (2) is hidden under the sheath (3) and a second position in which the collection thimble (23) is outside of the sheath (3).

5. The device according to the preceding claims, **characterized in that** the interior cannula (2) has a handle (22) on the proximal end thereof.

6. The device according to the preceding claims, **characterized in that** the sheath (3) has, in the proximal area, a gripping fin (33) for comfortable handling.

7. The device according to the preceding claims, **characterized in that** it comprises a protective cap (4) which covers the distal area of the sheath (3) and the collection thimble (23) of the interior cannula (2).

8. The device according to the preceding claims, **characterized in that** the interior cannula (2) and the sheath (3) are made of low-density polyethylene.

## Patentansprüche

1. Vorrichtung zur Selbstentnahme von Zellen aus dem Uterushals, die mindestens Folgendes umfasst:
- eine innere Kanüle (2), die an einem proximalen Ende einen Schieber (21) und an ihrem distalen Ende einen Entnahmekopf (23) aufweist; und
- eine äußere Hülle (3), durch welche die innere Kanüle (2) geführt wird, und
- Rotationsmittel (32) zwischen der inneren Kanüle (2) und der äußeren Hülle (3), die ein Drehen und Gleiten der äußeren Hülle (3) auf der inneren Kanüle (2) ermöglichen,
**dadurch gekennzeichnet, dass** die Rotationsmittel zwei Vorsprünge (24) auf gegenüberliegenden Seiten der Oberfläche des Kolbens (21) der Kanüle (2) aufweisen, die in entsprechende, an der Innenwand der Hülle (3) vorgesehene schraubenförmige Kanäle (32) passen

2. Vorrichtung nach Anspruch 1, **dadurch gekennzeichnet, dass** der Entnahmekopf (23) durch eine geprägte Oberfläche des distalen Endes des die innere Kanüle (2) bildenden Teils definiert ist.

3. Vorrichtung nach Anspruch 2, **dadurch gekennzeichnet, dass** die geprägte Oberfläche des Entnahmekopfs (23) eine gerillte Oberfläche ist, die durch parallele, an ihrem halbkugelförmigen Ende radial zusammenlaufende Längsrillen gebildet wird.

4. Vorrichtung nach den vorstehenden Ansprüchen, **dadurch gekennzeichnet, dass** die Rotationsmittel das Verschieben der äußeren Hülle (3) zwischen zwei Positionen ermöglichen: einer ersten Position, in der der Entnahmekopf (23) der Kanüle (2) in der Hülle (3) verdeckt liegt, und einer zweiten Position, in der sich der Entnahmekopf (23) außerhalb der Hülle (3) befindet.

5. Vorrichtung nach den vorstehenden Ansprüchen, **dadurch gekennzeichnet, dass** die innere Kanüle (2) an ihrem proximalen Ende einen Griff (22) aufweist.

6. Vorrichtung nach den vorstehenden Ansprüchen, **dadurch gekennzeichnet, dass** die Hülle (3) im proximalen Bereich eine Greifrippe (33) zur praktischen Handhabung aufweist.

7. Vorrichtung nach den vorstehenden Ansprüchen, **dadurch gekennzeichnet, dass** sie eine Schutzkappe (4) umfasst, die den distalen Bereich der Hülle (3) und den Entnahmekopf (23) der inneren Kanüle (2) bedeckt.

8. Vorrichtung nach den vorstehenden Ansprüchen, **dadurch gekennzeichnet, dass** die innere Kanüle (2) und die Hülle (3) aus Polyethylen niedriger Dichte bestehen.

## Revendications

1. Dispositif pour la collecte automatique de cellules du col de l'utérus, comprenant au moins
- une canule intérieure (2) qui comprend un piston (21) à une extrémité proximale et une douille collectrice (23) à l'extrémité distale de celle-ci ; et
- une gaine extérieure (3) qui est traversée par la canule intérieure (2), et
- des moyens de rotation (32) entre la canule intérieure (2) et la gaine extérieure (3) qui permettent la rotation et le glissement de la gaine extérieure (3) sur la canule intérieure (2),
**caractérisé en ce que** les moyens de rotation comprennent deux saillies (24) sur des côtés opposés de la surface du piston (21) de la canule (2) qui s'insèrent dans des canaux hélicoïdaux respectifs (32) prévus sur la paroi intérieure de la gaine (3)

2. Dispositif selon la revendication 1, **caractérisé en ce que** la douille collectrice (23) est défini par une surface gaufrée de l'extrémité distale de la pièce qui constitue la canule intérieure (2).

3. Dispositif selon la revendication 2, **caractérisé en ce que** la surface gaufrée de la douille collectrice (23) est une surface rainurée formée par des rainures longitudinales parallèles qui convergent radialement sur l'extrémité hémisphérique de celle-ci.

4. Dispositif selon les revendications précédentes, **caractérisé en ce que** les moyens de rotation permettent le déplacement de la gaine extérieure (3) entre deux positions, une première position dans laquelle la douille collectrice (23) de la canule (2) est cachée sous la gaine (3) et une seconde position dans laquelle la douille collectrice (23) est à l'extérieur de la gaine (3).

5. Dispositif selon les revendications précédentes, **caractérisé en ce que** la canule intérieure (2) a une poignée (22) sur l'extrémité proximale de celle-ci.

6. Dispositif selon les revendications précédentes, **caractérisé en ce que** la gaine (3) possède, dans la zone proximale, une ailette de préhension (33) pour une manipulation confortable.

7. Dispositif selon les revendications précédentes, **caractérisé en ce qu'**il comprend un capuchon de protection (4) qui couvre la zone distale de la gaine (3) et la douille collectrice (23) de la canule intérieure (2).

8. Dispositif selon les revendications précédentes, **caractérisé en ce que** la canule intérieure (2) et la gaine (3) sont fabriquées en polyéthylène de basse densité.
